# EUROPÄISCHE PATENTANMELDUNG

(11) **EP 4 458 981 A1**
(43) Veröffentlichungstag der Anmeldung: **06.11.2024**
(21) Anmeldenummer: 23171319.9
(22) Anmeldetag: 03.05.2023
(51) Int. Cl.: C12Q 1/18

(54) **INDIKATOR UND PRÜF-VERFAHREN ZUM BESTIMMEN EINER ANTI-MIKROBIELLEN UND/ODER ANTI-VIRALEN WIRKSAMKEIT EINER OBJEKT-OBERFLÄCHE EINES OBJEKTS UND COMPUTER-PROGRAMM-PRODUKT MIT EINEM DIGITALEN ZWILLING ZUR SIMULATION EINER FUNKTIONSFÄHIGKEIT DES INDIKATORS**

(71) Anmelder: Siemens Aktiengesellschaft, 80333 München (DE); Technische Universität Ilmenau, 98683 Ilmenau (DE)
(72) Erfinder: DOYE, Christian, 13156 Berlin (DE); JENSEN, Jens Dahl, 14050 Berlin (DE); WINKLER, Gabriele, 13587 Berlin (DE); GEBINOGA, Michael, 98693 Ilmenau (DE); GERHARD, Olivia, 98693 Ilmenau (DE); SCHOBER, Andreas, 99096 Erfurt (DE)
(74) Vertreter: Siemens Patent Attorneys

(57) **Zusammenfassung**

Die Erfindung betrifft einen Indikator zum Bestimmen einer anti-mikrobiellen und/oder anti-viralen Wirksamkeit einer Objekt-Oberfläche eines Objekts sowie ein entsprechendes Prüf-Verfahren. Dabei weist der Indikator mindestens einen Indikator-Träger mit mindestens einer Indikator-Oberfläche und die Indikator-Oberfläche mindestens einen Indikator-Farbstoff mit Farbstoff-Molekülen auf. Eine Indikator-Färbung des Indikator-Farbstoffs ist von einer Reaktion der Farbstoff-Moleküle des Indikator-Farbstoffs mit reaktiven Sauerstoff-Molekülen abhängig. Die Indikator-Oberfläche und die Objekt-Oberfläche können derart zusammengebracht werden, dass die Reaktion der Farbstoff-Moleküle des Indikator-Farbstoffs mit den reaktiven Sauerstoff-Molekülen der Objekt-Oberfläche ausgelöst werden kann. Aufgrund der durch die Reaktion der Farbstoff-Moleküle des Indikator-Farbstoffs mit den reaktiven Sauerstoff-Molekülen der Objekt-Oberfläche hervorgerufenen Indikator-Färbung des Indikator-Farbstoffs kann auf die anti-mikrobielle und/oder antivirale Wirksamkeit der Objekt-Oberfläche geschlossen werden. Vorzugsweise wird Methylenblau als Indikator-Farbstoff eingesetzt. Die Funktionsfähigkeit des Indikators wird mit Hilfe eines Computer-Programm-Produkts mit einem digitalen Zwilling simuliert.

## Beschreibung

Die Erfindung betrifft einen Indikator und ein Prüf-Verfahren zum Bestimmen einer anti-mikrobiellen und/oder anti-viralen Wirkung einer Objekt-Oberfläche eines Objekts. Darüber hinaus wird ein Computer-Programm-Produkt mit einem digitalen Zwilling zur Simulation einer Funktionsfähigkeit des Indikators angegeben.

Keime bilden als Mikro-Organismen - im Gegensatz zu Viren - vermehrungsfähiges biologisches Material. Um die Vermehrung von Keimen auf einer Objekt-Oberfläche eines Objekts zu unterbinden, kann die Objekt-Oberfläche selbst anti-mikrobiell wirken.

Bekannt ist der Nachweis einer anti-mikrobiellen Wirkung in flüssigen Medien. Dies gelingt über den Nachweis von reaktiven Sauerstoff-Verbindungen (reactive oxygen species, ROS). ROS sind hoch reaktive chemische Verbindungen, z.B. in Form von Sauerstoff-Radikalen. Der direkte Nachweis von ROS in flüssigen Medien erfolgt über gelöste (Farb-Indikatoren (z.B. Methylenblau, Allura Red, p-Nitrosodimethylanilin (PNDA)).

In biologischen Medien reagieren diese Indikatoren auf die Anwesenheit von ROS mittels einer Änderung einer Indikator-Färbung. Auf festen Oberflächen wird die Reaktion allerdings durch viele Stör-Faktoren so stark beeinflusst, dass eine fehlerfreie und damit belastbare Aussage erschwert bzw. nicht möglich ist. Zu diesen Stör-Faktoren zählen z.B. die Eigenfarbe der Objekt-Oberfläche, die die Beobachtung eines Farbumschlags der Indikator-Färbung erschwert. Zu den Stör-Faktoren zählen ebenso eine Geometrie und Beschaffenheit der Objekt-Oberfläche, z.B. eine Hydrophobie bzw. Hydrophilie der Objekt-Oberfläche.

Zum direkten Nachweis der anti-mikrobiellen Wirksamkeit einer Objekt-Oberfläche, dessen Wirkprinzip auf der Anwesenheit von reaktiven Sauerstoff-Molekülen basiert, ist derzeit kein Prüf-Verfahren bekannt, das ohne den Einsatz einer Keim-Lösung bzw. einem direkten Keim-Nachweisverfahren auskommt.

Aufgabe der vorliegenden Erfindung ist es, eine einfache Möglichkeit zum Überprüfen einer anti-mikrobiellen bzw. anti-viralen Wirksamkeit einer Objekt-Oberfläche eines Objekts anzugeben.

Zur Lösung der Aufgabe wird ein Indikator zum Bestimmen einer anti-mikrobiellen und/oder anti-viralen Wirksamkeit einer Objekt-Oberfläche eines Objekts angegeben. Dabei weist der Indikator mindestens einen Indikator-Träger mit mindestens einer Indikator-Oberfläche und die Indikator-Oberfläche mindestens einen Indikator-Farbstoff mit Farbstoff-Molekülen auf. Eine Indikator-Färbung des Indikator-Farbstoffs ist von einer Reaktion der Farbstoff-Moleküle des Indikator-Farbstoffs mit reaktiven Sauerstoff-Molekülen abhängig. Die Indikator-Oberfläche und die Objekt-Oberfläche können derart zusammengebracht werden, dass die Reaktion der Farbstoff-Moleküle des Indikator-Farbstoffs mit den reaktiven Sauerstoff-Molekülen der Objekt-Oberfläche ausgelöst werden kann. Aufgrund der durch die Reaktion der Farbstoff-Moleküle des Indikator-Farbstoffs mit den reaktiven Sauerstoff-Molekülen der Objekt-Oberfläche hervorgerufenen Indikator-Färbung des Indikator-Farbstoffs kann auf die anti-mikrobielle und/oder antivirale Wirksamkeit der Objekt-Oberfläche geschlossen werden.

Zur Lösung der Aufgabe wird auch ein Prüf-Verfahren zum Bestimmen einer anti-mikrobiellen und/oder anti-viralen Wirksamkeit einer Objekt-Oberfläche eines Objekts unter Verwendung des Indikators angegeben. Dabei werden die Objekt-Oberfläche des Objekts s und die Indikator-Oberfläche des Indikators in Kontakt gebracht, so dass auf der Objekt-Oberfläche des Objekts vorhandene reaktive Sauerstoff-Moleküle und Farbstoff-Moleküle des Indikator-Farbstoffs derart miteinander reagieren, dass eine Farbänderung der Indikator-Färbung hervorgerufen wird und somit die anti-mikrobielle und/oder anti-virale Wirksamkeit der Objekt-Oberfläche bestimmt werden kann.

Durch das In-Kontaktbringen der Objekt-Oberfläche und der Indikator-Oberfläche wird eine Reaktion von Indikator-Molekülen des Indikator-Farbstoffs mit den reaktiven Sauerstoff-Molekülen ausgelöst. Dadurch kommt es zu einer Änderung der Indikator-Färbung des Indikator-Farbstoffs. Damit wird die anti-mikrobielle und/oder anti-virale Wirkung der Objekt-Oberfläche sichtbar.

Gemäß einem weiteren Aspekt der Erfindung wird ein Computer-Programm-Produkt mit einem digitalen Zwilling zur Simulation einer Funktionsfähigkeit des Indikators angegeben. Mit Hilfe der Simulation wird die Funktionsfähigkeit bzw. eine Funktion des Indikators überprüft. Es wird überprüft, ob und wenn ja, wie wirksam der Indikator zum Bestimmen der anti-mikrobiellen und/oder anti-viralen Wirksamkeit der Objekt-Oberfläche des Objekts ist.

Das Objekt ist ein beliebiger fester (solider) Körper mit einer festen Objekt- bzw. Körper-Oberfläche. Als Grundlage der Erfindung dient die Reaktion von reaktiven Sauerstoff-Molekülen mit Indikator-Molekülen des Indikator-Farbstoffs. Die Reaktion führt zu einer Zersetzung der Farbstoff-Moleküle. In Folge davon kommt es zu einer Entfärbung des Farbstoffs.

Gemäß der Erfindung werden auf einer Objekt-Oberfläche gebildete reaktive Sauerstoff-Moleküle (z.B. Sauerstoff-Radikale) nachgewiesen. Neben diesem direkten, qualitativen Nachweis von reaktiven Sauerstoff-Molekülen ist es auch möglich, den Grad der anti-mikrobiellen Wirkung (anti-mikrobielle Wirksamkeit) der Objekt-Oberfläche zu bestimmen.

In einer besonderen Ausgestaltung ist die Indikator-Oberfläche des Indikator-Trägers von einer Indikator-Folie und/oder von einer Indikator-Platte gebildet. Der Indikator-Träger weist die Indikator-Folie und/oder die Indikator-Platte auf.

Der Indikator-Farbstoff kann auf beliebige Weise auf der Indikator-Oberfläche fixiert bzw. an die Indikator-Oberfläche gebunden sein. In einer besonderen Ausgestaltung ist der Indikator-Farbstoff mit Hilfe eines die Indikator-Oberfläche bildenden Hydrogels fixiert. Dabei sind die Farbstoff-Moleküle des Indikator-Farbstoffs in einer Matrix des Hydrogels verteilt. Folglich ist das Hydrogel entsprechend gefärbt. Die Verteilung der Farbstoff-Moleküle des Indikator-Farbstoffs in der Matrix des Hydrogels ermöglicht eine hohe Interaktion zwischen den reaktiven Sauerstoff-Molekülen und den Farbstoff-Molekülen des Indikator-Farbstoffs. Die reaktiven Sauerstoff-Moleküle auf der zu untersuchenden Objekt-Oberfläche können durch den Kontakt zwischen der Indikator-Oberfläche mit dem Hydrogel und der Objekt-Oberfläche durch die Matrix des Hydrogels wandern und Indikator-Moleküle des Indikator-Farbstoffs zersetzen. Dies führt zu einer lokalen Entfärbung des Hydrogels.

Das Hydrogel mit dem Indikator-Farbstoff zeichnet sich durch eine hohe Empfindlichkeit aus. Damit können reaktive Sauerstoff-Moleküle sehr schnell detektiert werden. Das liegt unter anderem an einem hohen Wasser-Anteil im Hydrogel, der eine Wechselwirkung zwischen den Farbstoff-Molekülen und den reaktiven Sauerstoff-Molekülen begünstigt.

Das Hydrogel ist vorzugsweise mit Hilfe einer Träger-Folie auf dem Indikator-Träger aufgetragen. Durch die Träger-Folie wird das Hydrogel stabilisiert. Die Träger-Folie fungiert als Applikations-Hilfe. Dadurch ist ein unkompliziertes und sicheres Auflegen bzw. Abziehen des Hydrogels von der Oberfläche ermöglicht.

Als Indikator-Farbstoffe sind verschiedenste Farbstoffe denkbar, die eine Reaktion mit reaktiven Sauerstoff-Molekülen eingehen. Dabei kann nur eine Art Indikator-Farbstoff vorgesehen sein. Mischungen mehrerer Indikator-Farbstoffe sind ebenso denkbar.

Geeignete Indikator-Farbstoffe sind Allura Red und p-Nitrosodimethylanilin (PNDA). Ein besonders geeigneter Indikator-Farbstoff ist Methylenblau. In einer besonderen Ausgestaltung weist daher der Indikator-Farbstoff Methylenblau auf. Durch die Reaktion von Methylenblau-Molekülen mit reaktiven Sauerstoff-Molekülen verlieren die Methylenblau-Moleküle ihre ursprüngliche blaue Farbe. Sie werden entfärbt.

Gemäß einer besonderen Ausgestaltung wird zum Bestimmen der anti-mikrobiellen und/oder anti-viralen Wirksamkeit der Objekt-Oberfläche ein Bestimmen einer anti-mikrobiellen und/oder anti-viralen Aktivität der Objekt-Oberfläche durchgeführt. Die anti-mikrobielle und/oder anti-virale Wirksamkeit der Objekt-Oberfläche wird damit nicht nur qualitativ, sondern auch quantitativ erfasst.

In einer besonderen Ausgestaltung wird zum Bestimmen der anti-mikrobiellen und/oder antiviralen Wirksamkeit und/oder zum Bestimmen der anti-mikrobiellen und/oder antiviralen Aktivität eine spektroskopische Untersuchung der Indikator-Färbung des Indikator-Farbstoffs durchgeführt. Die Indikator-Färbung des Indikator-Farbstoffs wird spektroskopisch untersucht. Mit der spektroskopischen Untersuchung können die anti-mikrobiellen Wirksamkeit bzw. die anti-mikrobielle Aktivität genau erfasst werden. Bevorzugt kommen hier Absorptions- und/oder Transmission-Spektroskopie im ultravioletten (UV) oder im sichtbaren (Vis) Wellenlängenbereich zum Einsatz.

Mit dem Prüf-Verfahren kann nicht nur die anti-mikrobielle bzw. anti-virale Wirksamkeit der Objekt-Oberfläche bestimmt werden. Es kann auch eine Kontamination der Objekt-Oberfläche mit Keimen festgestellt werden. Gemäß einer besonderen Ausgestaltung wird daher mit Hilfe der bestimmten anti-mikrobiellen Wirksamkeit und/oder mit Hilfe der bestimmten anti-mikrobiellen Aktivität auf die Anwesenheit von Keimen auf der Objekt-Oberfläche geschlossen. Die Keim-Belastung der Objekt-Oberfläche wird ermittelt. Auf der Objekt-Oberfläche vorhandene Keime können direkt nachgewiesen werden. Der Nachweis der Keime gelingt dabei keim- bzw. kulturunabhängig.

Zusammenfassend sind mit der Erfindung folgende Vorteile verbunden:
- Mit der Erfindung ist es möglich, die anti-mikrobielle Wirksamkeit einer festen Objekt-Oberfläche eines beliebigen Objekts einfach und schnell zu bestimmen.
- Aufgrund der unspezifischen Wirksamkeit der reaktiven Sauerstoff-Verbindungen kann mit der anti-mikrobiellen Wirksamkeit auch eine anti-virale Wirksamkeit der Objekt-Oberfläche bestimmt werden.
- Reaktive Sauerstoff-Verbindungen auf der Objekt-Oberfläche können qualitativ und quantitativ erfasst werden.
- Der Einsatz der Erfindung ist ungefährlich.
- Die Überprüfung der anti-mikrobiellen und/oder anti-viralen Wirksamkeit der Objekt-Oberfläche gelingt ohne Veränderung oder gar Zerstörung der Objekt-Oberfläche.
- Der Indikator belastet die Umwelt nicht und könnte mit dem Hausmüll entsorgt werden.
- Insbesondere bei Verwendung von Methylenblau als Indikator-Farbstoff sind fehlerfreie und damit belastbare Aussagen über
die anti-mikrobielle bzw. anti-virale Wirksamkeit der Objekt-Oberfläche möglich.
- Die anti-mikrobielle Wirksamkeit der Objekt-Oberfläche kann kulturunabhängig bestimmt werden.
- Die eingangs beschriebenen Störfaktoren, wie sie bei der herkömmlichen Bestimmung der antimikrobiellen Wirksamkeit von Objekt-Oberflächen auftreten können, spielen hier keine Rolle.

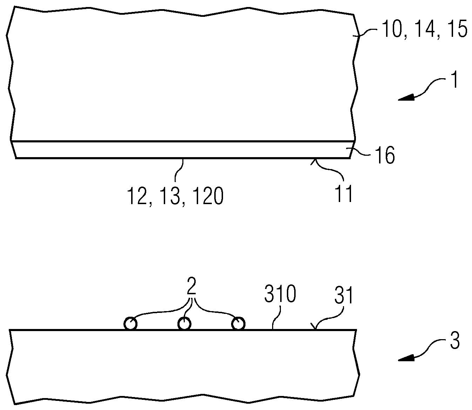

Anhand eines Ausführungsbeispiels und der dazugehörigen Figur wird die Erfindung näher beschrieben. Die Figur ist schematisch und stellt keine maßstabsgetreue Abbildung dar.

Die Figur zeigt im Querschnitt einen Indikator und ein Objekt, deren Objekt-Oberfläche auf deren anti-mikrobielle und/oder anti-virale Wirksamkeit hin untersucht werden soll.

Gegeben ist ein Indikator 1 zum Bestimmen einer anti-mikrobiellen und/oder anti-viralen Wirksamkeit einer Objekt-Oberfläche 31 eines Objekts 3.

Der Indikator 1 weist einen Indikator-Träger 10 mit einer Indikator-Oberfläche 11 auf. Die Indikator-Oberfläche 11 weist einen Indikator-Farbstoff 12 auf. Dabei ist eine Indikator-Färbung 13 des Indikator-Farbstoffs 12 von einer Reaktion von Indikator-Molekülen des Indikator-Farbstoffs 12 mit reaktiven Sauerstoff-Molekülen abhängig. Als Indikator-Farbstoff kommt Methylenblau zum Einsatz.

Die Indikator-Oberfläche 11 und die Objekt-Oberfläche 31 können derart zusammengebracht werden, dass die Reaktion der Farbstoff-Moleküle 120 des Indikator-Farbstoffs 12 mit den reaktiven Sauerstoff-Molekülen 310 der Objekt-Oberfläche 31 ausgelöst werden kann. Aufgrund der durch die Reaktion der Farbstoff-Moleküle 120 des Indikator-Farbstoffs 12 mit den reaktiven Sauerstoff-Molekülen 310 der Objekt-Oberfläche 31 hervorgerufenen Indikator-Färbung 13 des Indikator-Farbstoffs 12 kann auf die anti-mikrobielle und/oder antivirale Wirksamkeit der Objekt-Oberfläche 31 geschlossen werden.

Mit Hilfe des Indikators 1 wird ein Prüf-Verfahren zum Bestimmen einer anti-mikrobiellen und/oder anti-viralen Aktivität der Objekt-Oberfläche 31 des Objekts 3 durchgeführt. Dazu werden die Objekt-Oberfläche 31 des Objekts 3 und die Indikator-Oberfläche 10 des Indikator-Trägers 10 in Kontakt gebracht, so dass auf der Objekt-Oberfläche 31 des Objekts 3 vorhandene reaktive Sauerstoff-Moleküle 310 und Farbstoff-Moleküle 120 des Indikator-Farbstoffs 12 derart miteinander reagieren, dass eine Farbänderung der Indikator-Färbung 13 hervorgerufen wird und somit die anti-mikrobielle und/oder anti-virale Wirksamkeit bzw. Aktivität der Objekt-Oberfläche 31 bestimmt werden kann.

Zum Bestimmen der anti-mikrobiellen und/oder antiviralen Wirksamkeit bzw. zum Bestimmen der anti-mikrobiellen und/oder antiviralen Aktivität wird eine spektroskopische Untersuchung der Indikator-Färbung 13 des Indikator-Farbstoffs 12 durchgeführt. Dazu wird Absorptions-Spektroskopie im sichtbaren Wellenlängenbereich des elektromagnetischen Spektrums eingesetzt.

Gemäß einem weiteren Anwendungsfall wird mit Hilfe der bestimmten anti-mikrobiellen Wirksamkeit bzw. Aktivität auf die Anwesenheit von Keimen 2 auf der Objekt-Oberfläche 31 geschlossen.

Die Funktionsfähigkeit des Indikators 1 wird mit Hilfe eines Computer-Programm-Produkts mit einem digitalen Zwilling simuliert.

## Patentansprüche

1. Indikator (1) zum Bestimmen einer anti-mikrobiellen und/oder anti-viralen Wirksamkeit einer Objekt-Oberfläche (31) eines Objekts (3),
wobei
- der Indikator (1) mindestens einen Indikator-Träger (10) mit mindestens einer Indikator-Oberfläche (11) aufweist,
- die Indikator-Oberfläche (11) mindestens einen Indikator-Farbstoff (12) mit Farbstoff-Molekülen (120) aufweist,
- eine Indikator-Färbung (13) des Indikator-Farbstoffs (12) von einer Reaktion der Farbstoff-Moleküle (120) des Indikator-Farbstoffs (12) mit reaktiven Sauerstoff-Molekülen (310) abhängig ist und
- die Indikator-Oberfläche (11) und die Objekt-Oberfläche (31) derart zusammengebracht werden können, dass die Reaktion der Farbstoff-Moleküle (120) des Indikator-Farbstoffs (12) mit den reaktiven Sauerstoff-Molekülen (310) der Objekt-Oberfläche (31) ausgelöst werden kann und
- aufgrund der durch die Reaktion der Farbstoff-Moleküle (120) des Indikator-Farbstoffs (12) mit den reaktiven Sauerstoff-Molekülen (310) der Objekt-Oberfläche (31) hervorgerufenen Indikator-Färbung (13) des Indikator-Farbstoffs (12) auf die anti-mikrobielle und/oder antivirale Wirksamkeit der Objekt-Oberfläche (31) geschlossen werden kann.

2. Indikator nach Anspruch 1, wobei die Indikator-Oberfläche (11) des Indikator-Trägers (10) von einer Indikator-Folie (14) und/oder von einer Indikator-Platte (15) gebildet ist.

3. Indikator nach Anspruch 1 oder 2, wobei der Indikator-Farbstoff (12) mit Hilfe eines die Indikator-Oberfläche (11) bildenden Hydrogels (16) fixiert ist.

4. Indikator nach Anspruch 4, wobei das Hydrogel (16) mit Hilfe einer Träger-Folie zur Bildung der Indikator-Oberfläche (11) auf dem Indikator-Träger (10) aufgetragen ist.

5. Indikator nach einem der Ansprüche 1 bis 4, wobei der Indikator-Farbstoff (12) Methylenblau aufweist.

6. Prüf-Verfahren zum Bestimmen einer anti-mikrobiellen und/oder anti-viralen Wirksamkeit einer Objekt-Oberfläche (31) eines Objekts (3) unter Verwendung eines Indikators (1) nach einem der Ansprüche 1 bis 5, wobei die Objekt-Oberfläche (31) des Objekts (3) und die Indikator-Oberfläche (11) des Indikator-Trägers (10) in Kontakt gebracht werden, so dass auf der Objekt-Oberfläche (31) des Objekts(3) vorhandene reaktive Sauerstoff-Moleküle (310) und Farbstoff-Moleküle (120) des Indikator-Farbstoffs (12) derart miteinander reagieren, dass eine Farbänderung der Indikator-Färbung (13) hervorgerufen wird und somit die anti-mikrobielle und/oder anti-virale Wirksamkeit der Objekt-Oberfläche (31) bestimmt werden kann.

7. Prüf-Verfahren nach Anspruch 6, wobei zum Bestimmen der anti-mikrobiellen und/oder anti-viralen Wirksamkeit der Objekt-Oberfläche (11) ein Bestimmen einer anti-mikrobiellen und/oder anti-viralen Aktivität der Objekt-Oberfläche (11) durchgeführt wird.

8. Prüf-Verfahren nach Anspruch 6 oder 7, wobei zum Bestimmen der anti-mikrobiellen und/oder antiviralen Wirksamkeit und/oder zum Bestimmen der anti-mikrobiellen und/oder antiviralen Aktivität eine spektroskopische Untersuchung der Indikator-Färbung (13) des Indikator-Farbstoffs (12) durchgeführt wird.

9. Prüf-Verfahren nach einem der Ansprüche 6 bis 8, wobei mit Hilfe der bestimmten anti-mikrobiellen Wirksamkeit und/oder mit Hilfe der bestimmten anti-mikrobiellen Aktivität auf die Anwesenheit von Keimen (2) auf der Objekt-Oberfläche (31) geschlossen wird.

10. Computer-Programm-Produkt mit einem digitalen Zwilling zur Simulation einer Funktionsfähigkeit eines Indikators (1) nach einem der Ansprüche 1 bis 5.
